# EUROPEAN PATENT APPLICATION

(11) **EP 4 331 361 A1**
(43) Date of publication of application: **06.03.2024**
(21) Application number: 22796179.4
(22) Date of filing: 28.04.2022
(51) Int. Cl.: A01N 1/02, C12N 1/04, C12N 5/00

(54) **FREEZE-DRY PROTECTIVE AGENT FOR EXTRACELLULAR VESICLES**

(30) Priority: 28.04.2021 KR 20210055116
(71) Applicant: Incheon National University Research & Business Foundation, Incheon 22012 (KR); S&E Bio Co., Ltd., Seoul 06351 (KR)
(72) Inventor: CHA, Jae Min, Incheon 22009 (KR); KANG, Wu Young, Bucheon-si Gyeonggi-do 14541 (KR); KIM, Eun Hee, Seoul 06608 (KR); SHIN, Eun Kyoung, Seoul 05855 (KR); BAN, Jae Bok, Guri-si Gyeonggi-do 11914 (KR)
(74) Representative: Cabinet Becker et Associés
(86) International application number: PCT/KR2022/006105
(87) International publication number: WO 2022/231347

(57) **Abstract**

The present invention relates to a composition, for freeze-dry protecting extracellular vesicles, capable of preserving the function and characteristics of extracellular vesicles, and an extracellular vesicle freeze-drying method using same. Trehalose and sucrose of the present invention are added to an extracellular vesicle freeze-drying process to solve the problems of crystal growth and redissolution capacity degradation, which are problems in an existing freeze-dry protective agent, and also maintain the unique marker, functional factor expressions and therapeutic effects of the extracellular vesicles, and thus may be variously utilized for producing various therapeutic agents using extracellular vesicles.

## Description

### [Technical Field]

The present invention relates to a composition for freeze-dry protecting extracellular vesicles, capable of preserving the functions and characteristics of extracellular vesicles, and an extracellular vesicle freeze-drying method using the same.

### [Background Art]

Treatments using stem cells, particularly mesenchymal stem cells (MSCs), and positive clinical results have been reported for various diseases. However, a stem cell therapeutic agent has a risk of cell-related side effects such as vascular occlusion, tumor formation, and coagulation disorders, and efficacy verification through clinical trials is still required. It is known that a paracrine effect of stem cells induces the promotion of the regeneration and vascular regeneration of surrounding skin cells, and in particular, extracellular vesicles (EV) are known as a major efficacious factor in the paracrine effect.

The extracellular vesicles are classified into exosomes and microvesicles depending on a size, and the exosomes have a diameter of 30 to 150 nm, and the microvesicles have a size of 100 to 1,000 nm. The extracellular vesicles are parts of the cell membrane that have been separated into the blood, and are known to mediate intercellular communication by containing both protein and nuclear components. Using extracellular vesicles instead of stem cells not only increases safety by minimizing the side effects by using stem cells, but also is advantageous in terms of biodistribution and production process.

However, despite the usefulness of extracellular vesicles, there is still a lack of research on technologies and stable formulations for a method capable of isolating and then stably maintaining and storing extracellular vesicles, which are difficult to obtain.

Therefore, there is a need for a new preservation method to stably maintain and store extracellular vesicles that can be used as therapeutic agents for various diseases.

### [Disclosure]

### [Technical Problem]

Therefore, the present inventors have studied technology for stably maintaining and storing extracellular vesicles, identified a new freeze-dry protective agent capable of effectively improving problems that may occur during a freeze-drying process and preserving unique properties and functions of extracellular vesicles, and then completed the present invention.

Accordingly, an object of the present invention is to provide a composition for freeze-dry protecting extracellular vesicles including trehalose and sucrose, and a method for freeze-drying extracellular vesicles using the same.

### [Technical Solution]

In order to achieve the object, the present invention provides a composition for freeze-dry protecting extracellular vesicles including trehalose and sucrose.

The present invention also provides a freeze-dry protective agent for extracellular vesicles, including trehalose and sucrose.

In addition, the present invention provides a composition for freeze-drying extracellular vesicles including a mixture of trehalose and sucrose as a freeze-dry protective agent; and extracellular vesicles.

In addition, the present invention provides a freeze-drying method of extracellular vesicles including 1) mixing a mixture of trehalose and sucrose, as a freeze-dry protective agent, with extracellular vesicles; and 2) freeze-drying the mixture in step 1).

### [Advantageous Effects]

According to the present invention, trehalose and sucrose are added to an extracellular vesicle freeze-drying process to solve the problems of crystal growth and redissolution capacity degradation, which are problems in an existing freeze-dry protective agent, and also maintain the unique marker, functional factor expressions and therapeutic effects of the extracellular vesicles, and thus may be variously utilized for producing various therapeutic agents using extracellular vesicles.

### [Description of Drawings]

FIG. 1 is a diagram illustrating a result of visually identifying extracellular vesicles freeze-dried and rehydrated by adding DMSO, trehalose, and mannitol as a freeze-dry protective agent.
FIG. 2 is a diagram illustrating a result of confirming the number (A) and size distribution (B) of extracellular vesicles (EV) obtained through qNano measurement after treating, freeze-drying and then rehydrating trehalose and mannitol as a freeze-dry protective agent.
FIGS. 3A and 3B are diagrams illustrating results of confirming nanoparticle formation through XRD peak analysis after freeze-drying only PBS alone, and mannitol (A) and trehalose (B) diluted in PBS without extracellular vesicles (EV).
FIG. 3C is a diagram illustrating a result of confirming through qNano analysis unspecified nanoparticles produced in an experimental group in which PBS and 0.07 or 0.17% of trehalose (T) were freeze-dried and then redissolved (Lyo: freeze-drying experimental group, -80: freezing and redissolving experimental group at -80°C). FIG. 4D is a diagram illustrating a result of confirming the redissolution of unspecified nanoparticles formed after freeze-drying PBS and 0.07% of trehalose (T) at room temperature for 48 hours.
FIG. 4A is a diagram illustrating a result of confirming through qNano measurement unspecified nanoparticles produced in a trehalose (T) alone experimental group after freeze-drying and redissolution and a trehalose and sucrose (T + S)-mixed freeze-drying and redissolution experimental group without extracellular vesicles (EV) (*p < 0.05, **p < 0.01, ***p < 0.001).
FIG. 4B is a diagram illustrating a result of confirming redissolution and dissolution rates of unspecified nanoparticles produced in a trehalose (T) alone experimental group after freeze-drying and redissolution and a trehalose and sucrose (T + S)-mixed freeze-drying and redissolution experimental group without extracellular vesicles (EV) for 20 minutes.
FIG. 4C is a diagram illustrating a result of confirming through XRD analysis crystals produced in a trehalose (T) alone experimental group after freeze-drying and redissolution and a trehalose and sucrose (T + S)-mixed freeze-drying and redissolution experimental group without extracellular vesicles (EV).
FIG. 5 is a diagram illustrating a result of confirming the number of EV particles in extracellular vesicles (EV) freeze-dried and redissolved after treating a freeze-dry protective agent mixed with 2 to 8% trehalose and sucrose.
FIG. 6 is a diagram illustrating a result of confirming changes in size distribution of extracellular vesicles (EV) freeze-dried and re-hydrated after treating a freeze-dry protective agent mixed with trehalose and sucrose.
FIG. 7 is a diagram illustrating a result of confirming the EV shape of each experimental group in Cryo-EM data.
FIG. 8 is a diagram illustrating a result of confirming changes in total protein amount in extracellular vesicles (EV) freeze-dried and re-hydrated after treating a freeze-dry protective agent mixed with 2 to 8% trehalose and sucrose (*p < 0.05, **p < 0.01, ***p < 0.001).
FIG. 9 is a diagram illustrating a result of confirming changes in the amount of total RNA and the expression amount of miRNA related to treatment efficacy in extracellular vesicles (EV) freeze-dried and re-hydrated after treating a freeze-dry protective agent mixed with 2 to 8% trehalose and sucrose.
FIG. 10 is a diagram illustrating a result of confirming expression of an EV marker CD63 in extracellular vesicles (EV) freeze-dried and re-hydrated after treating a freeze-dry protective agent (TS) mixed with 2 to 8% trehalose and sucrose (*p < 0.05, **p < 0.01, ***p < 0.001).
FIG. 11 is a diagram illustrating a result of confirming a blood vessel regeneration effect through tube forming capacity and cell migration capacity in extracellular vesicles (EV) freeze-dried and re-hydrated after treating PBS, VEGF, Fresh EV and a freeze-dry protective agent (TS) mixed with trehalose and sucrose (*p < 0.05, **p < 0.01, ***p < 0.001).

### [Best Mode for the Invention]

The present invention provides a composition for freeze-dry protecting extracellular vesicles including trehalose and sucrose, and a method for freeze-drying extracellular vesicles using the same.

According to the present invention, the composition for freeze-dry protecting extracellular vesicles may solve the problems of crystal growth and redissolution capacity degradation, which are problems in an existing freeze-dry protective agent, and also maintain or increase the unique marker, functional factor expressions and therapeutic effects of the extracellular vesicles.

Hereinafter, the present invention will be described in detail.

The present invention provides a composition for freeze-dry protecting extracellular vesicles including trehalose and sucrose and an extracellular vesicle freeze-dry protective agent including trehalose and sucrose, which is characterized by including trehalose and sucrose as active ingredients as an extracellular vesicle freeze-dry protective agent. As such, when trehalose and sucrose are mixed and treated to extracellular vesicles, it is possible to not only solve the problems of forming unidentified nanoparticles where single ingredients such as trehalose, mannitol, and sucrose form salts and crystals by a PBS component during the freeze-drying process, but also to increase the dissolution rate when redissolving the freeze-dried extracellular vesicles.

Accordingly, the present invention provides a composition for freeze-drying extracellular vesicles including a mixture of trehalose and sucrose as a freeze-dry protective agent; and extracellular vesicles.

The trehalose and the sucrose may be 1 to 10% (w/v), preferably 2 to 9% (w/v), more preferably 2 to 8% (w/v) of trehalose and sucrose, respectively. When less than 1 % (w/v) of trehalose or sucrose is used, there may be a problem of generating a large number of unidentified nanoparticle crystals, and the redissolution rate may also be slow.

The present invention is characterized by mixing and adding trehalose and sucrose as active ingredients of a freeze-dry protective agent, and the trehalose and the sucrose may be mixed in a volume ratio of preferably 1 : 0.05 to 5, more preferably 1 : 0.5 to 3, and even more preferably 1 : 0.5 to 2. In a preferred embodiment of the present invention, the effect was confirmed using a freeze-dry protective agent mixed in a 1 : 1 volume ratio.

The freeze-dry protective composition of the present invention may include a mixture of 1 to 10% (w/v) trehalose and sucrose mixed in a volume ratio of 1 : 0.05 to 5, and may include a mixture of trehalose and sucrose at the concentrations in a volume ratio of 1 : 0.1 to 3, more preferably 1 : 0.5 to 1.

The freeze-dry protective agent composition mixed with trehalose and sucrose of the present invention may reduce crystal formation that occurs during freeze-drying, rehydration, and redissolution of extracellular vesicles.

The trehalose and sucrose as the freeze-dry protective agent may be mixed with extracellular vesicles to be freeze-dried in a volume ratio of 1 : 0.5 to 5, preferably 1 : 0.5 to 3, more preferably 1 : 0.5 to 2.

The freeze-dry protective agent is mixed with the extracellular vesicles, frozen at -70 to -90°C, and freeze-dried in a freeze-dryer for 3 to 7 days to produce the freeze-dried extracellular vesicles.

The freeze-dry protective agent is diluted with PBS as a base and prepared, and may use sterilized distilled water for redissolution after freeze-drying. Through this process, the compounds constituting freeze-dried PBS together with extracellular vesicles are dissolved in distilled water again, and the extracellular vesicles may be preserved in PBS.

The extracellular vesicles to be freeze-dried of the present invention may be extracellular vesicles isolated from cells isolated from natural organisms or extracellular vesicles isolated from ingredients such as milk. In addition, the cells may be derived from any type of animal or plant, including humans and non-human mammals, and may be various types of immune cells, tumor cells, and stem cells, and preferably, the stem cells may be mesenchymal stem cells, pluripotent stem cells, induced pluripotent stem cells, or embryonic stem cells.

The extracellular vesicles refer to vesicles released outside the cells and include microvesicles, apoptotic vesicles, exosomes, etc. Therefore, the present invention may include, without limitation, various extracellular vesicles capable of achieving the purpose by adding trehalose and sucrose as freeze-dry protective agents, and for example, the extracellular vesicles may be exosomes.

The exosomes of the present invention mean all vehicles (e.g., exosome-like vehicles) that have a nano-sized vesicle structure that is secreted from cells of various animals, plants, bacteria, fungi, and algae and released into an extracellular space, and have compositions similar to exosomes. In particular, the exosome of the present invention may be exosomes derived from stem cells.

In the present invention, the extracellular vesicles may be extracellular vesicles that exhibit therapeutically useful effects, and may be treated with the freeze-dry protective agent composition and the freeze-dry protective agent of the present invention during the freeze-drying process of these extracellular vesicles, so that unique characteristics, properties, and therapeutic effects of the extracellular vesicles may be maintained at equivalent levels or higher.

In the present invention, when freeze-drying the extracellular vesicles by adding trehalose and sucrose, it was confirmed that the formation of undesired and unidentified nanoparticle crystals was suppressed during the freeze-drying process, but the rehydration and dissolution rates thereof were significantly accelerated. Furthermore, it was confirmed that there was no loss in the amount of total protein and the amount of total RNA contained in the extracellular vesicles, and the levels of miRNA exhibiting therapeutic effect were also maintained equally. In addition, it was confirmed that CD63, a marker for extracellular vesicles, also exhibited a similar or increased expression pattern compared to before freeze-drying.

The therapeutic efficacy of the extracellular vesicles, whose efficacy is maintained by the freeze-dry protective agent, may be a broad regenerative or immunomodulatory effect, which is the inherent therapeutic efficacy of extracellular vesicles, and for example, may be blood vessel regeneration capacity. The extracellular vesicles frozen by the freeze-dry protective agent of the present invention may be used as pharmaceutical compositions, cosmetic compositions, food compositions, and external skin compositions, and may be administered orally or parenterally.

The types of diseases requiring angiogenesis to which the freeze-dried extracellular vesicles of the present invention may be applied are not limited thereto, but may be at least one selected from the group consisting of burns, ulcers, ischemia, arteriosclerosis, angina pectoris, myocardial infarction, cardiovascular diseases, cerebrovascular diseases, and alopecia, particularly cardiovascular diseases or cerebrovascular diseases.

The pharmaceutical composition including the freeze-dried extracellular vesicles of the present invention may further include appropriate carriers, excipients, and diluents commonly used in the production of pharmaceutical compositions, in addition to the active ingredients. The pharmaceutical composition of the present invention may further include other pharmaceutically active ingredients or active mixtures.

The pharmaceutical composition of the present invention may be formulated and used in the form of oral formulations such as powders, granules, tablets, capsules, suspensions, emulsions, syrups, and aerosols, external preparations, suppositories, and sterile injectable solutions according to conventional methods, respectively. The carrier, the excipient, and the diluent that may be included in the composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. When the pharmaceutical composition is formulated, the formulation may be prepared by using diluents or excipients, such as a filler, an extender, a binder, a wetting agent, a disintegrating agent, and a surfactant, which are generally used. Solid formulations for oral administration include tablets, pills, powders, granules, capsules, and the like, and these solid formulations may be prepared by mixing at least one or more excipients, for example, starch, calcium carbonate, sucrose or lactose, gelatin, and the like. Further, lubricants such as magnesium stearate and talc are used in addition to simple excipients.

Liquid formulations for oral administration may correspond to suspensions, oral liquids, emulsions, syrups, and the like, and may include various excipients, for example, a wetting agent, a sweetener, an aromatic agent, a preservative, and the like, in addition to water and liquid paraffin which are commonly used as simple diluents. Formulations for parenteral administration include sterilized aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, and the like may be used. As a base compound of the suppository, witepsol, macrogol, tween 61, cacao butter, laurinum, glycerogelatin, and the like may be used.

A preferable dose of the pharmaceutical composition of the present invention varies according to the condition and weight of a patient, the degree of a disease, a drug form, and the route and period of administration, but may be properly selected by those skilled in the art. The administration may be performed once a day or several times a day. The dose does not limit the scope of the present invention in any aspect.

The pharmaceutical composition of the present invention may be administered to mammals such as mice, rats, livestock, and humans through various routes. All methods of administration may be expected, and for example, the pharmaceutical composition may be administered by oral, rectal or intravenous, intramuscular, subcutaneous, intrauterine dural or intracerebroventricular injection.

The definitions of terms for the excipients, binders, disintegrants, lubricants, flavor enhancers, flavoring agents, and the like of the present invention are described in literature known in the art and include those with the same or similar functions.

Further, the present invention provides a freeze-drying method of extracellular vesicles including 1) mixing a mixture of trehalose and sucrose, as freeze-dry protective agents, with extracellular vesicles; and 2) freeze-drying the mixture in step 1).

The description of the freeze-drying method may be applied in the same manner as the description of the freeze-dry protective composition and the freeze-dry protective agent described above.

In the freeze-drying method, the freeze-drying of step 2) may include freezing at -70 to -90°C and then freeze-drying for 3 to 7 days. The freezing may be performed overnight, and the freeze-drying may be performed under a pressure of 1 to 7 mTorr, and may be performed in first, second, or third freeze-drying processes.

Duplicated contents are omitted in consideration of the complexity of the present specification, and terms not defined otherwise in the present specification have the meanings commonly used in the art to which the present invention pertains.

Terms not defined otherwise in this specification have meanings commonly used in the technical art to which the present invention pertains.

Hereinafter, the present invention will be described in detail by Examples. However, the following Examples are just illustrative of the present invention, and the contents of the present invention are not limited to the following Examples.

### [Modes for the Invention]

### Example 1. Cell culture and collection of extracellular vesicles

Human bone marrow-derived stem cells were cultured to collect exosomes, which were extracellular vesicles (hereinafter, EV) to be used in an experiment. The cells were incubated at a concentration of 2.5 × 10⁵ cells in a 100 mm culture dish (SPL, 20100), and incubated in a low-glucose Dulbecco's modified Eagle's medium (DMEM, Life Technologies Corporation, CA, USA) filtered to 0.2 µm and a medium of 10% fetal bovine serum (FBS, Life Technologies Corporation) and 1% antibioticsantimycotics (Life Technologies Corporation). Subculture was performed every 80 to 90% from P2 to P6, and incubated in an incubator at 37°C and 5% CO₂.

Stem cells incubated under the same conditions after P6 were incubated for 5 days in a low-glucose DMEM, and a medium consisting of 10% exosome-depleted fetal bovine serum (System Biosciences, Palo Alto, CA, USA) and 1% antibioticsantimycotics. Thereafter, the culture medium was harvested and centrifuged under conditions of 4°C, 2500 g, and 10 min, and only a supernatant was taken and filtered to 0.2 µm. The medium collected above was immediately subjected to tangential flow filtration (TFF), and subjected to concentration and diafiltration, respectively, and concentrated at about 10 times the starting volume. At this time, the membrane was used with a Minimate TFF 300K membrane (Pall Corporation, NY, USA). Exosomes were collected through the same process as above, and used in subsequent freeze-drying experiments.

### Example 2. Freeze-drying treatment

To perform freeze-drying of EVs, EVs were collected as in Example 1, and then the size and concentration were measured by qNano (IZON Ltd, Christchurch, New Zealand) using tunable resistive pulse sensing technology. Thereafter, the freeze-dry protective agent and exosomes were mixed at a 1 : 1 volume ratio so that the final concentration was 1 × 10¹¹ EVs/ml. As candidates for selecting a freeze-dry protective agent suitable for exosomes, trehalose (Sigma, St. Louis, MO, USA), mannitol (Sigma), DMSO (Sigma), and sucrose (Sigma) were used.

As an experimental group, mannitol 0 to 5% (w/v), DMSO 0 to 10% (w/v), and trehalose 0 to 4% (w/v) were set as a single-administered group; and a mixture of sucrose and trehalose at each concentration of 2 to 8% (w/v) mixed in a 1 : 1 volume ratio was used as the experimental group. After mixing, the mixture was kept overnight at -80°C and then freeze-dried (-80°C, 5 mTorr) for 4 days. After freeze-drying was completed, the mixture was sealed and stored at room temperature. The sample stored above was subjected to a rehydration process for use in the experiment, and upon rehydration, deionized water filtered to 0.2 µm was added to the original volume of the mixture and kept at 37°C for 30 minutes.

### Example 3. Visual analysis after freeze-drying

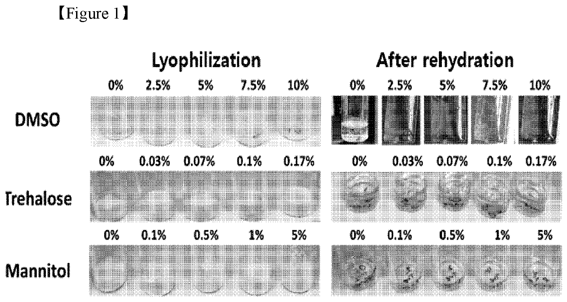

Exosomes were mixed while changing the freeze-dry protective agent to 0 to 10% DMSO, 0.1 to 5% mannitol, and 0.03 to 0.17% trehalose, and the results of visually confirming the frozen state and the state after rehydration were illustrated in FIG. 1.

As illustrated in FIG. 1, in the case of trehalose and mannitol, it was confirmed that freeze-drying had progressed due to the presence of fine particles after freeze-drying upon visual observation, but in the case of DMSO, it was confirmed that thin film-like materials were formed after freeze-drying, so that DMSO was unsuitable as a freeze-drying agent for EVs. In particular, it was confirmed visually that the DMSO-mixed group was not dissolved in PBS well and large particles were present. On the other hand, it was confirmed that the mixed group of trehalose and mannitol was well redissolved visually even after rehydration.

### Example 4. Analysis of EV concentration and size

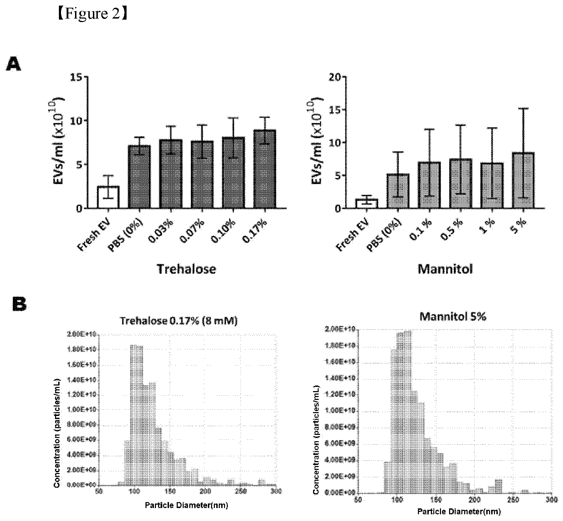

As in Example 2, trehalose and mannitol were treated as a freezing protective agent and frozen, and then rehydrated, and the number and size of EVs obtained were confirmed through qNano measurement. qNano was a device for measuring the concentration and size of particles through tunable resistive pulse sensing technology. A nanopore used in the experiment was NP200, the radial stretch was 46.5 mm, a voltage of 0.66 mV was applied, and the concentration and size of EVs in the sample were measured using 0.075 ml of the sample. All the samples were analyzed by measuring at least 500 particles, and all of the samples were calibrated with CPC100 (100mm, 1.4E + 13 particles/ml, IZON Ltd). The measurement result was illustrated in FIG. 2. Meanwhile, since the DMSO-treated group was not dissolved well in PBS, qNano measurement was not possible.

As illustrated in FIG. 2, even though the same number of EVs as a Fresh EV group, which was an experimental group where qNano was measured immediately after EV isolation, was mixed with a freeze-dry protective agent and rehydrated, and the number of EVs was measured, it was determined that the number of nanoparticles was increased in both the trehalose and mannitol-treated groups and the PBS-treated group. This means that a large number of unidentified nanoparticles other than EVs were additionally produced, and as a result of nanoparticle size distribution analysis of qNano, it was confirmed that the unidentified nanoparticles had sizes similar to EVs, in the range of 80 to 300 nm.

### Example 5. XRD analysis of EVs after treatment with freeze-dry protective agent

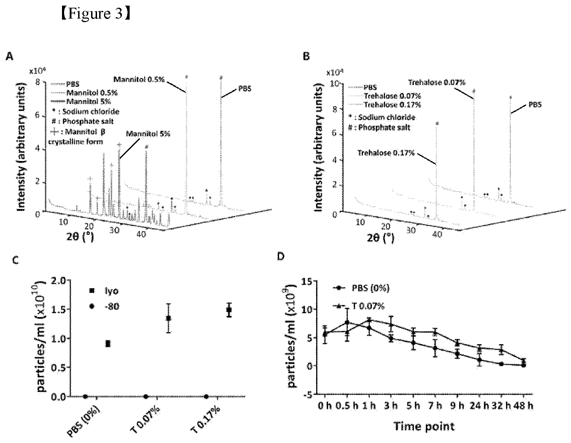

It was known that a freeze-dry protective agent may form salts and crystals due to the presence of a PBS component, and that crystallization may be promoted under certain conditions. In order to confirm newly generated unidentified nanoparticles other than EVs as confirmed in Example 4, only PBS alone, and mannitol and trehalose diluted in PBS were freeze-dried without EVs, and whether nanoparticles were formed after freeze-drying was confirmed through XRD peak analysis. The internal structure was analyzed by measuring XRD under Cu radiation (45 kV × 200 mA) using Smartlab (Rigaku, JP). The range of 2θ was set to 2 to 45, and the analysis was performed by setting the step size to 0.05° and the dwell time to 2 sec, and the results were illustrated in FIG. 3. In XRD peak analysis, peaks at 22 to 23° and 27 to 28° indicated crystals of sodium chloride by the PBS component, and peaks at 32 to 33° meant crystals of phosphate.

As illustrated in FIG. 3, as XRD peak analysis results of an experimental group freeze-dried by adding mannitol (FIG. 3A), even in addition to the salt crystals caused by the PBS component, a plurality of peaks was generated, which indicated that the crystals themselves were formed by the mannitol component. Mannitol was known as a crystalline freezing protective agent and had three types of crystals. It was known that β-mannitol crystals, one of the representative crystal forms among the types, were stable crystals, which were not dissolved well in water. Meanwhile, in the case of trehalose (FIG. 3B), it was confirmed that only peaks for sodium chloride crystals and phosphate crystals by the PBS component were formed.

FIG. 3C is a diagram illustrating a qNano analysis result of confirming that unspecified nanoparticles that did not exist before were formed when only PBS or a trehalose solution without EV was freeze-dried and then redissolved. In an experimental group that was thawed after freezing without freeze-drying at -80°C (indicated by -80 in FIG. 3C), it was confirmed that the PBS and the trehalose solution were redissolved, so that these nanoparticles were not formed, and the particles were not measured in qNano measurement. Meanwhile, in a 0.07% trehalose experimental group (2 mM) or a 0.17% trehalose experimental group (8 mM), which was a lowconcentration trehalose-added experimental group, more particles were formed than when freeze-drying with PBS alone.

As the XRD analysis result, it was assumed that the unspecified nanoparticles generated during freeze-drying of the trehalose/PBS solution were sodium chloride and phosphate, so that it was confirmed whether the sodium chloride and phosphate may be gradually dissolved over time. As a result of redissolving the sodium chloride and phosphate while shaking at room temperature, as confirmed in FIG. 3D, it was confirmed that almost all of the salts were redissolved after about 48 hours.

When described these results together, it was confirmed that when trehalose was used as a freeze-dry protective agent at a low concentration, crystallized salts were generated during the freeze-drying process, which took a long time for redissolution.

### Example 6. Crystallinity reduction experiment by treatment with freeze-dry protective agent

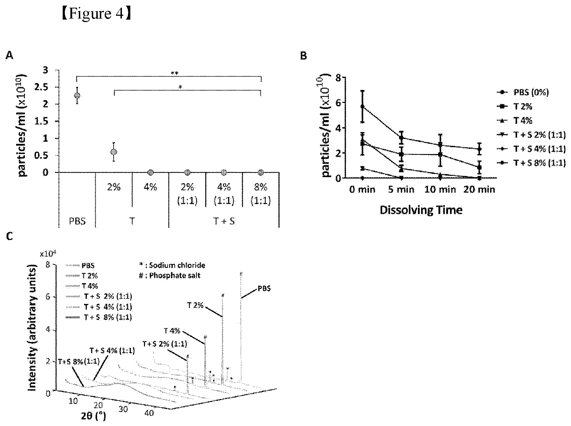

In Example 5, when a low concentration of trehalose was used as a freeze-dry protective agent, a problem of generating salt crystals was confirmed, and thus, in order to solve the problem, the formation of nanoparticles was confirmed in an experimental group in which the concentration of trehalose was changed from 2 to 4%, and in an experimental group in which trehalose and sucrose, known as an amorphous freezing protective agent were mixed. 2 to 8% (w/v) of trehalose and sucrose were mixed at a 1 : 1 volume ratio and used as a freeze-dry protective agent experimental group. Using the experimental group, a freeze-dry protective agent and PBS were mixed and redissolved without EV, and qNano analysis and XRD analysis were performed in the same manner as Examples 4 and 5. The results were illustrated in FIG. 4.

As illustrated in FIG. 4A, as a result of qNano measurement immediately after freeze-drying and redissolution (within 3 minutes), it was confirmed that a few nanoparticles were measured in a 2% trehalose group compared to a group treated with PBS alone, and in a group treated with 4% trehalose and a mixture of 1 : 1 trehalose and sucrose, it was confirmed that no nanoparticles were measured by being completely redissolved.

In FIG. 4B, the dissolution rate according to redissolution time was confirmed, and it was confirmed that the higher the concentration of the freeze-dry protective agent, the faster the dissolution rate. In particular, the mixed solution of trehalose and sucrose showed very excellent dissolution capacity as redissolution was achieved within 5 minutes.

As illustrated in FIG. 4C, as the concentration of the freeze-drying protective agent increased, the XRD analysis peaks also decreased, and in particular, it was confirmed that the trehalose and sucrose-mixed solution had a very low degree of crystal formation.

Through the results, it was confirmed that as the concentration increased, crystal generation and redissolution capacity increased, and in particular, the trehalose and sucrose mixture had a significantly low crystal generation rate and excellent redissolution rate during freeze-drying.

As these results, when freeze-drying EVs using a mixture of trehalose and sucrose as a freeze-dry protective agent, unidentified nanoparticle crystals were not formed or quickly redissolved, and thus, the mixture was suitable as a freeze-dry protective agent for an EV therapeutic agent required to be administered to patients by controlling the number of EVs.

### Example 7. Confirmation of crystal formation inhibition effect of freeze-dry protective agent mixed with trehalose and sucrose

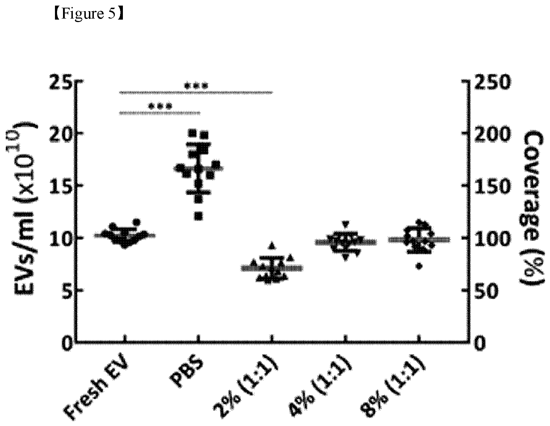

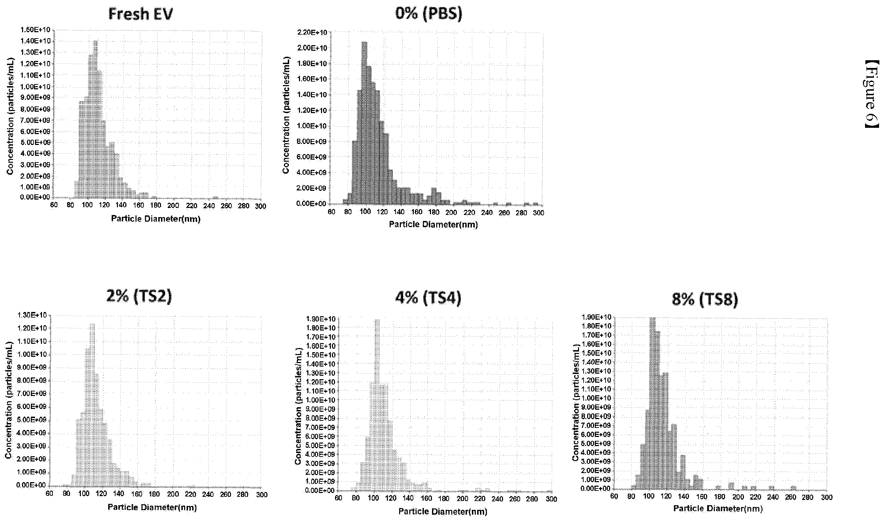

Since it has been confirmed that the freeze-dry protective agent mixed with trehalose and sucrose was suitable as a freeze-dry protective agent for an EV therapeutic agent, when applied to freeze-drying of the MSC-EVs obtained in Example 1, the redissolution rate was further confirmed, and the results were illustrated in FIGS. 5 and 6. As experimental groups, an experimental group in which EVs were treated with a freeze-dry protective agent mixed with 2, 4, and 8% (w/v) of trehalose and sucrose in a 1 : 1 volume ratio in the same manner as in Example 2. and freeze-dried and redissolved, an experimental group in which EVs were freeze-dried and redissolved in PBS without a freeze-dry protective agent (0%), and a Fresh EV, which was EV immediately after isolation of EVs that were not freeze-dried, were used.

The EVs were treated with the freeze-dry protective agent and then freeze-dried and redissolved, and the number of EV particles was confirmed, and the results were illustrated in FIG. 5.

As illustrated in FIG. 5, when redissolved after freeze-drying, there was no statistically significant difference in the trehalose and sucrose-mixed group at a concentration of 2%, but a slight decrease in the number of nanoparticles was confirmed compared to Fresh EV. On the other hand, in 4 and 8% experimental groups, it was confirmed that almost 100% of the initial number of freeze-dried EVs were preserved and redissolved.

When redissolved after freeze-drying, changes in EV size were confirmed, and the results were illustrated in FIG. 6.

As illustrated in FIG. 6, in the experimental group added with a freeze-dry protective agent, a size distribution similar to that of Fresh EV was shown, but in an experimental group added with PBS alone, nanoparticles of rather small sizes were identified.

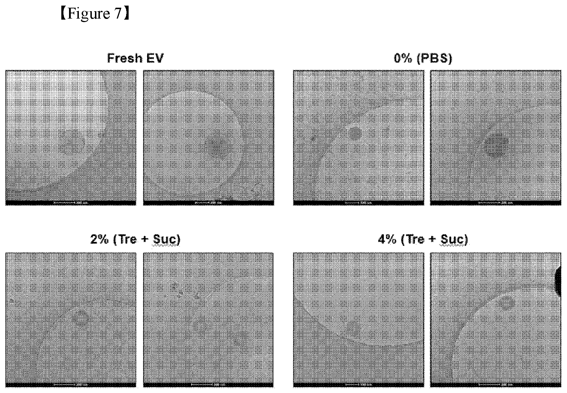

In addition, the shape of EV particles in each experimental group was confirmed through Cryo-EM. The Fresh EV experimental group was able to be used immediately, but the freeze-dried experimental group was redissolved at 37°C for 30 minutes and then used in the experiment. Before sampling, a Glow discharge system (PELCO easiGlow^{™}, Ted pella) was used to make grids (Quantifoil, R1.2/1.3, 200 mesh, EMS) into a hydrophilic surface. 4 µl of each sample was placed on the grid and blot force 3 was applied for 1.5 seconds while maintaining 100% moisture at 4°C. Thereafter, the samples were frozen in liquid ethane using a Vitrobot Mark IV (FEI) method to vitrify the samples. Thereafter, analysis was performed at the Nanobioimaging center (Seoul national university, Korea) using a Talos L120C (FEI) microscope with gun type Lab6 at 120 kV. The results were illustrated in FIG. 7.

As illustrated in FIG. 7, in EVs freeze-dried and rehydrated using only PBS without the freeze-dry protective agent of the present invention, many fine particles presumed to be salts were observed in the background, but it was confirmed that fine particles were almost not observed in EVs using the freeze-dry protective agent mixed with trehalose and sucrose.

The results showed that in the freeze-dried group using PBS, nanoparticles were confirmed about twice greater than the initial freeze-dried EVs, and new nanoparticles out of the EV size distribution were identified, and thus there was a crystal formation problem as confirmed above. On the other hand, when the mixture of trehalose and sucrose was treated as the freeze-dry protective agent, there was no problem of crystal formation, and thus, it was confirmed that EVs may be obtained by redissolving after freeze-drying while preserving the number of EVs.

### Example 8. Confirmation of effect on EVs of freeze-dry protective agent mixed with trehalose and sucrose

In order to be used as a freeze-dry protective agent for an EV therapeutic agent, it is important not only to obtain EVs without EV loss or production of other materials after freeze-drying and redissolution, but also not to affect the properties and functions of EVs to be freeze-dried. Accordingly, in EVs treated with the mixture of trehalose and sucrose as the freeze-dry protective agent and frozen and redissolved, it was confirmed whether the total RNA contained in EVs was restored without loss and whether changes in miRNA, which was an efficacy factor for EVs, were not induced, and changes in the expression of CD63 as an EV marker were confirmed.

Protein quantitative analysis was performed as follows: All the samples were centrifuged at 120000 g for 1 hour using an ultra-high-speed centrifuge (Beckman Coulter, CA, USA), and then the supernatant was discarded, and lysis was performed. The lysis was performed using RIPA (Life Technologies Corporation), and proteins were quantified using BCA protein assay (Life Technologies Corporation).

Analysis of CD63, the EV marker, was confirmed through ELISA and was performed using the following method: ELISA was performed with a commercial kit according to the manual of a manufacturer. A CD63 (EH95RB, ThermoFisher Scientific, Inc, Waltham, MA, USA) ELISA kit included standard proteins, and thus the amounts of proteins and extracellular vesicles were determined based on the standard curve of the kit. Isolated Fresh EV and freeze-dried EVs were dispensed in the same amount (200 uL/well) of EVs and Standard in a 96-well microplate coated with a capture antibody without pretreatment, and then reacted at 4°C. The next day, a sandwich-type ELISA method was performed and the absorbance was measured using a microplate reader.

Analysis of the expression of miRNA, which was an efficacy factor of EV, was performed by qPCR and preceded as follows: RNA was extracted from EVs using Trizol^{™} according to the instructions of the manufacturer and RNA was quantified by a nanodrop. RNA was made into cDNA through a reverse transcription (RT) process, and real-time PCR was performed according to the manual of the manufacturer using Taqman probes suitable for miRNA and mRNA.

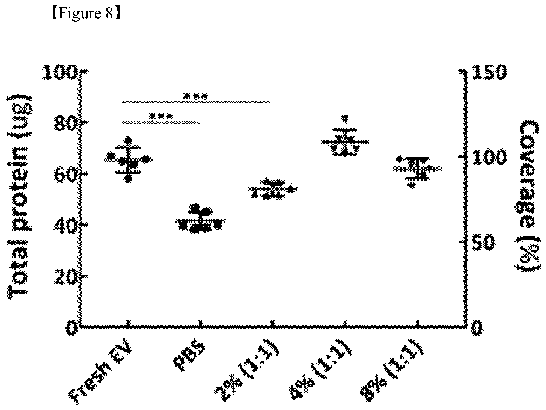

The results of confirming changes in EV protein amount, total RNA recovery rate, miRNA amount, and EV marker expression as described above were illustrated in FIGS. 8 to 10.

As illustrated in FIG. 8, a PBS-treated group showed a decrease in protein amount by about 38% compared to Fresh EV, but in an experimental group treated with the mixture of trehalose and sucrose of the present invention as a freeze-dry protective agent, there was a small decrease in protein, especially in the 4 and 8% mixture groups, protein amounts similar to that in the Fresh EV group were measured.

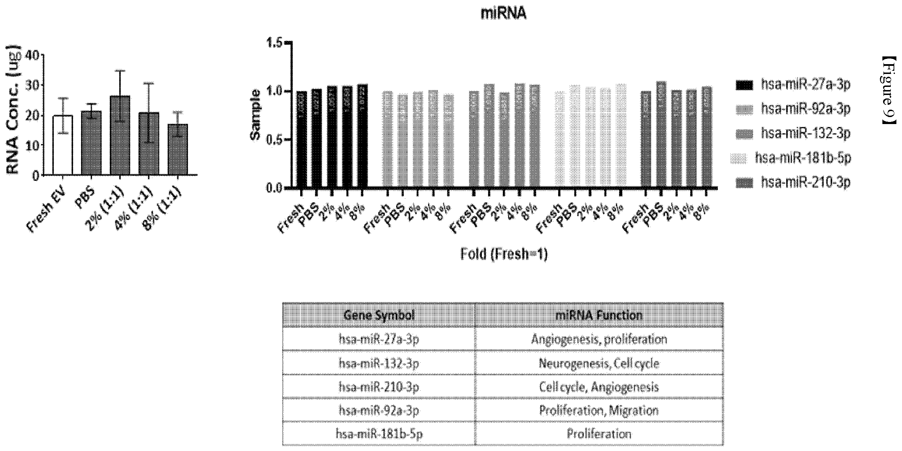

As illustrated in FIG. 9, the total RNA recovery rate upon redissolution after freeze-drying did not show a statistically significant difference in all experimental groups, and miRNA with therapeutic efficacy was also expressed uniformly in all experimental groups without significant differences.

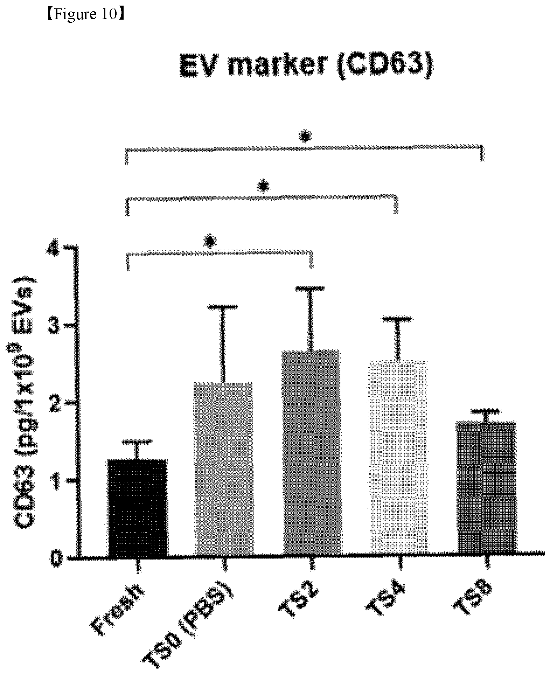

As illustrated in FIG. 10, there was no significant difference in the expression of CD63 as the EV marker, between respective experimental groups.

### Example 9. Verification of blood vessel regeneration capacity of frozen and thawed EVs after treatment with freeze-dry protective agent

EV was known to have blood vessel regeneration capacity and be able to be used as a therapeutic agent for various diseases requiring angiogenesis. An experiment was performed to determine whether EVs mixed with the freeze-dry protective agent of the present invention, and then frozen and redissolved, could maintain the inherent blood vessel regeneration capacity of EVs to an equivalent degree or improve the effect. The EV blood vessel regeneration capacity was confirmed through tube formation capacity and cell migration capacity experiments using HUVEC cells. More specifically, in order to see the effect of neovascularization by EVs obtained in Example 1, HUVECs attached on Matrigel were treated with EVs to confirm the tube formation effect. Specifically, HUVECs were cultured in an M199 medium (Gibco) supplemented with 20% FBS, 5 U/mL heparin, and 3 ng/mL bFGF. The cells were inoculated on a growth factor-reduced Matrigel Matrix (BD Bioscience, MA, USA) from µ-Slides Angiogenesis (ibidi, Graefelfing, Germany) at a density of 1.0 × 10⁴ cells, and the tube was formed for 7 hours in a humidifying chamber under a 37°C and 5% CO₂ environment. Images were taken on a phase-contrast microscope (Olympus), and the number of tubular structures was quantified in a microscope field (4× magnification) using ImageJ software.

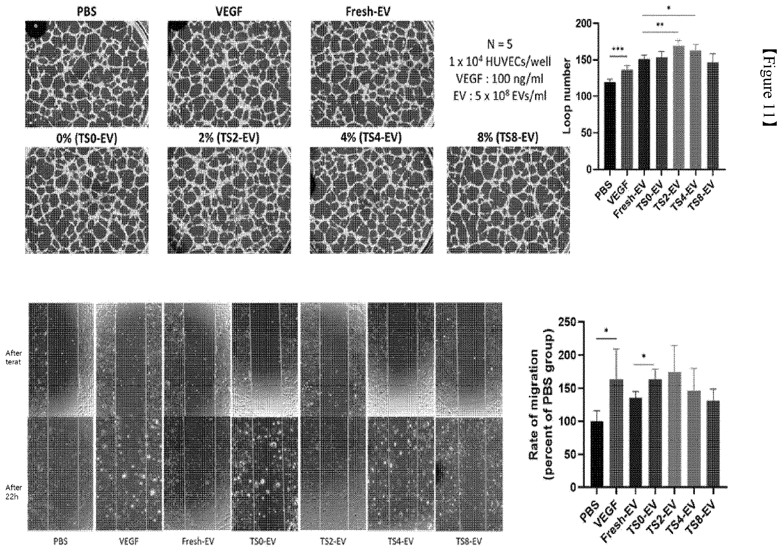

For analysis of cell migration capacity, HUVECs were incubated in a M199 medium (Gibco) supplemented with 20% FBS, 5 U/mL heparin, and 3 ng/mL bFGF in a humidifying chamber with 37°C and 5% CO₂ environment. 1.0 × 10⁵ cells were cultured in a 24-well plate to reach 100% confluency and then treated with MMC (2.5 ug/ml) to stop cell division. The cells were scratched vertically using a pipette tip and then washed with PBS. The scratches were imaged under a phase-contrast microscope (Olympus) and the corresponding regions were marked. The same area of the scratched wound was photographed again after 7 hours, and the degree of cell migration and wound closure was measured using ImageJ software. As freeze-dry protective agent experimental groups, an EV experimental group freeze-dried and rehydrated by treating a freeze-dry protective agent mixed with 0, 2, 4, and 8% (w/v) trehalose and sucrose (freeze-dried and rehydrated after treatment with PBS) in a 1 : 1 volume, Fresh EV, which was EV immediately after isolation without treatment with the freeze-dry protective agent, a positive control VEGF-treated group and a PBS-treated group. As a negative control, an experimental group in which HUVECs were treated with PBS was used. EVs were all treated at 5 × 10⁸ EV/ml, and treated with VEGF at 100 ng/ml. The result of confirming angiogenesis capacity was illustrated in FIG. 11.

As illustrated in FIG. 11, all EV-treated groups showed a similar level of blood vessel regeneration effect to the VEGF-treated experimental group, and particularly, in an experimental group treated with EVs frozen and redissolved by treating a 1 : 1 mixture of trehalose and sucrose at a concentration of 2 and 4% as a freeze-dry protective agent, excellent blood vessel regeneration capacity was confirmed. This result shows that the therapeutic efficacy of EVs may be maintained and restored without loss when treating and freeze-drying and then redissolving the freeze-dry protective agent of the present invention.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A composition for freeze-dry protecting extracellular vesicles, comprising trehalose and sucrose.

2. The composition for freeze-dry protecting extracellular vesicles of claim 1, wherein the trehalose and the sucrose are contained in 1 to 10% (w/v), respectively.

3. The composition for freeze-dry protecting extracellular vesicles of claim 1, wherein the trehalose and the sucrose are mixed and contained in a volume ratio of 1 : 0.05 to 5.

4. The composition for freeze-dry protecting extracellular vesicles of claim 1, wherein the trehalose and the sucrose reduce crystal formation during freeze-drying and rehydrating of the extracellular vesicles.

5. The composition for freeze-dry protecting extracellular vesicles of claim 1, wherein the composition is mixed with extracellular vesicles in a volume ratio of 1 : 0.5 to 5.

6. The composition for freeze-dry protecting extracellular vesicles of claim 1, wherein the extracellular vesicles are exosomes or microvesicles.

7. The composition for freeze-dry protecting extracellular vesicles of claim 1, wherein the extracellular vesicles are extracellular vesicles derived from immune cells, tumor cells, or stem cells.

8. An extracellular vesicle freeze-dry protective agent, comprising trehalose and sucrose.

9. A composition for freeze-drying extracellular vesicles comprising:
a mixture of trehalose and sucrose as a freeze-dry protective agent; and
extracellular vesicles.

10. The composition for freeze-drying extracellular vesicles of claim 9, wherein the extracellular vesicles have blood vessel regeneration capacity.

11. A freeze-drying method of extracellular vesicles comprising:
1) mixing a mixture of trehalose and sucrose as a freeze-dry protective agent with extracellular vesicles; and
2) freeze-drying the mixture of step 1) above.

12. The freeze-drying method of extracellular vesicles of claim 11, wherein step 2) includes freezing the mixture of step 1) at -70 to -90°C and then freeze-drying the mixture for 3 to 7 days.
